# EUROPÄISCHE PATENTANMELDUNG

(11) **EP 4 036 933 A1**
(43) Veröffentlichungstag der Anmeldung: **03.08.2022**
(21) Anmeldenummer: 21154501.7
(22) Anmeldetag: 01.02.2021
(51) Int. Cl.: G16H 50/70, G16H 70/40

(54) **KLASSIFIZIERUNG VON MITTEILUNGEN ÜBER ARZNEIMITTEL**

(71) Anmelder: Bayer AG, 51373 Leverkusen (DE)
(72) Erfinder: DIETRICH, Jürgen, 13467 Berlin (DE)
(74) Vertreter: BIP Patents

(57) **Zusammenfassung**

Die vorliegende Erfindung befasst sich mit dem technischen Gebiet der Analyse von Mitteilungen über Arzneimittel, deren Wirkungen und/oder Nebenwirkungen.

## Beschreibung

Die vorliegende Erfindung befasst sich mit dem technischen Gebiet der Analyse von Mitteilungen über Arzneimittel, deren Wirkungen und/oder Nebenwirkungen.

Unter dem Begriff Pharmakovigilanz werden alle Aktivitäten zusammengefasst, die sich mit der Erfassung, Bewertung, dem Verstehen und der Prävention von Nebenwirkungen von Arzneimitteln befassen.

Dabei spielt die Erfassung von so genannten unerwünschten Ereignissen (englisch: *adverse events*) eine wichtige Rolle. Bei einem unerwünschten Ereignis handelt es sich um einen unerwünschten Vorfall, der im Rahmen einer interventionellen oder nicht-interventionellen Studie oder im Rahmen einer Therapie zu einem Arzneimittel bei einem Patienten bzw. einer Versuchsperson auftritt.

Darüber hinaus ist es in der Pharmakovigilanz wichtig, Ursache und Wirkung bei Arzneimittelanwendungen und zusätzliche Informationen zum Anwendungsfall zu identifizieren. Die Schwierigkeit liegt darin, dass diese Informationen die gleiche medizinische Terminologie verwenden.

Es gibt folgende Identifizierungsmerkmale:
- Arzneimittelnebenwirkung
- zugelassener Anwendungsfall eines Arzneimittels (therapeutische Indikation oder kurz: Indikation) entsprechend der Fachinformation des Arzneimittels (*Summary of Product Characteristics,* SmPC); das ist in der Regel eine Krankheit, ein Symptom oder eine Prozedur.
- der beabsichtigte Effekt eines Arzneimittels; diese Information ist Bestandteil der Indikation und beschreibt das Resultat der Anwendung, z.B. Prophylaxe, Palliativtherapie, Kuration, Prävention.
- Ursache der Krankheit (z.B. steroid-induzierte Osteoporose)
- Zielgruppe (z.B. postmenopausale Osteoporose)
- Begleiterkrankungen, die zusätzlich zu einer Grunderkrankung vorliegen können (z.B. als Abgrenzung zur Indikation: Behandlung von Pankreasinsuffizienz bei Patienten mit Mukoviszidose, die Begleiterkrankung ist hier Mukoviszidose)

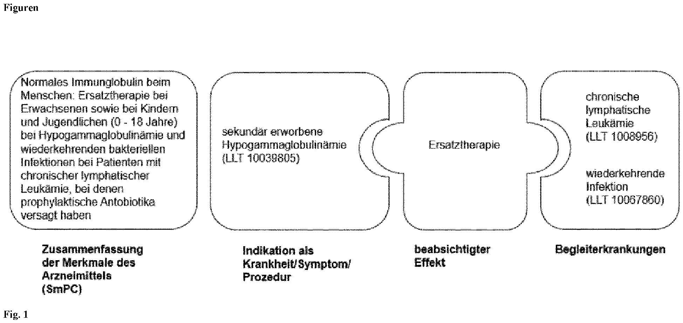

Fig. 1 zeigt die Beziehungen zwischen den Identifizierungsmerkmalen schematisch an einem Beispiel.

Es gibt zahlreiche Publikationen, die sich mit der automatisierten Erfassung, Analyse und/oder Bewertung von unerwünschten Ereignissen befassen (siehe z.B. EP2755155A2, WO2017/021368A1, US20200226218A1).

Es besteht jedoch nicht nur ein Interesse an der Identifizierung von unerwünschten Ereignissen von Arzneimitteln. Auch positive Reaktionen auf eine Therapie mit einem Arzneimittel können von Interesse sein. Eine positive Reaktion kann beispielsweise, insbesondere wenn sie unerwartet ist, auf eine neue Anwendungsmöglichkeit des Arzneimittels hinweisen. Viele Arzneimittel besitzen noch nicht bekannte positive Reaktionen, die in der Vergangenheit meist nur durch Zufall erkannt wurden (z.B. Viagra, Digoxin).

Die vorliegende Erfindung widmet sich dem Aspekt der Klassifizierung von Arzneimittelinformationen.

Ein erster Gegenstand der vorliegenden Erfindung ist ein computer-implementiertes Verfahren umfassend die Schritte:
- Empfangen einer Mitteilung über einen Gesundheitszustand einer Person und/oder über eine Änderung eines Gesundheitszustands der Person und/oder über eine Wirkung eines Arzneimittels und/oder über ein Ausbleiben einer Wirkung des Arzneimittels und/oder über eine Therapie
- Einordnen der Mitteilung in eine von mindestens zwei Klassen,
   ∘ wobei die mindestens zwei Klassen eine erste Klasse umfassen,
   ∘ wobei die Mitteilung in die erste Klasse eingeordnet wird, wenn die Mitteilung eine Aussage über eine Verbesserung des Gesundheitszustands der Person enthält und/oder wenn die Mitteilung eine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird,
- für den Fall, dass die Mitteilung in die erste Klasse eingeordnet wurde: Ausgeben der Mitteilung und/oder Übertragen der Mitteilung und/oder Speichern der Mitteilung.

Ein weiterer Gegenstand der vorliegenden Erfindung ist ein Computersystem, umfassend
- eine Eingabeeinheit,
- eine Steuer- und Recheneinheit und
- eine Ausgabeeinheit,
   - wobei die Steuer- und Recheneinheit konfiguriert ist, die Eingabeeinheit zu veranlassen, eine Mitteilung zu empfangen, wobei die Mitteilung eine Aussage über einen Gesundheitszustand einer Person und/oder über eine Änderung eines Gesundheitszustands der Person und/oder über eine Wirkung eines Arzneimittels und/oder über ein Ausbleiben einer Wirkung des Arzneimittels und/oder über eine Therapie umfasst
   - wobei die Steuer- und Recheneinheit konfiguriert ist, die Mitteilung in eine von mindestens zwei Klassen einzuordnen, eine erste Klasse und eine zweite Klasse, wobei die Mitteilung in die erste Klasse eingeordnet wird, wenn die Mitteilung eine Aussage über eine Verbesserung des Gesundheitszustands der Person enthält und/oder wenn die Mitteilung eine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird,
   - wobei die Steuer- und Recheneinheit konfiguriert ist, die Ausgabeeinheit zu veranlassen, die Mitteilung auszugeben und/oder zu übertragen und/oder zu speichern, wenn die Mitteilung in die erste Klasse eingeordnet wurde.

Ein weiterer Gegenstand der vorliegenden Erfindung ist ein computerlesbares Speichermedium, umfassend Befehle, die bei der Ausführung durch ein Computersystem dieses veranlassen, die folgenden Schritte auszuführen:
- Empfangen einer Mitteilung über einen Gesundheitszustand einer Person und/oder über eine Änderung eines Gesundheitszustands der Person und/oder über eine Wirkung eines Arzneimittels und/oder über ein Ausbleiben einer Wirkung des Arzneimittels und/oder über eine Therapie
- Einordnen der Mitteilung in eine von mindestens zwei Klassen,
   ∘ wobei die mindestens zwei Klassen eine erste Klasse umfassen,
   ∘ wobei die Mitteilung in die erste Klasse eingeordnet wird, wenn die Mitteilung eine Aussage über eine Verbesserung des Gesundheitszustands der Person enthält und/oder wenn die Mitteilung eine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird,
- für den Fall, dass die Mitteilung in die erste Klasse eingeordnet wurde: Ausgeben der Mitteilung und/oder Übertragen der Mitteilung und/oder Speichern der Mitteilung.

Die Erfindung wird nachstehend näher erläutert, ohne zwischen den Erfindungsgegenständen (Verfahren, Computersystem, Speichermedium) zu unterscheiden. Die nachfolgenden Erläuterungen sollen vielmehr für alle Erfindungsgegenstände in analoger Weise gelten, unabhängig davon, in welchem Kontext (Verfahren, Computersystem, Speichermedium) sie erfolgen.

Wenn in der vorliegenden Beschreibung oder in den Patentansprüchen Schritte in einer Reihenfolge genannt werden, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden können; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die vorliegende Erfindung stellt Mittel bereit, die es erlauben, Mitteilungen über den (geänderten) Gesundheitszustand von Personen und/oder über Reaktionen von Personen auf einen oder mehrere Arzneimittel automatisiert zu analysieren, um insbesondere diejenigen Mitteilungen zu identifizieren, die eine positive Aussage über den (geänderten) Gesundheitszustand einer Person und/oder eine positive Reaktion einer Personen auf einen oder mehrere Arzneimittel und/oder eine Therapie enthalten.

Eine "Person" im Sinne der vorliegenden Erfindung ist vorzugsweise ein Mensch, der aufgrund einer bestehenden Erkrankung oder prophylaktisch ein oder mehrere Arzneimittel erhält und/oder einnimmt. Dabei soll der Begriff "Einnahme" nicht auf eine orale Verabreichung beschränkt sein, sondern alle Applikationsformen einschließen. Synonym zum Begriff "Person" wird in dieser Beschreibung auch der Begriff "Patient" verwendet.

"Arzneimittel", auch als "Arzneistoffe" bezeichnet, sind Stoffe oder Zubereitungen aus Stoffen, die zur Anwendung im oder am menschlichen oder tierischen Körper bestimmt sind und als Mittel mit Eigenschaften zur Heilung oder Linderung oder zur Verhütung menschlicher oder tierischer Krankheiten oder krankhafter Beschwerden bestimmt sind, oder die im oder am menschlichen oder tierischen Körper angewendet oder einem Menschen oder einem Tier verabreicht werden können, um entweder die physiologischen Funktionen durch eine pharmakologische, immunologische oder metabolische Wirkung wiederherzustellen, zu korrigieren oder zu beeinflussen oder eine medizinische Diagnose zu erstellen. Unter dem Begriff "Arzneimittel" wird in dieser Beschreibung insbesondere der Wirkstoff oder die Kombination von Wirkstoffen verstanden, der/die für die Wirksamkeit eines Medikaments verantwortlich ist.

Ein "Medikament" ist ein Arzneimittel, das in bestimmter Dosierung zur Heilung, Vorbeugung oder Diagnose einer Krankheit dient.

In einer Ausführungsform der vorliegenden Erfindung sollen die Begriffe "Arzneimittel" und "Medikament" auch Prüfpräparate umfassen, für die noch keine behördliche Arzneimittelzulassung vorliegt.

In einem ersten Schritt wird eine Mitteilung empfangen. Üblicherweise wird eine Vielzahl an Mitteilungen empfangen, die parallel oder sequenziell analysiert und klassifiziert werden.

Eine "Mitteilung" im Sinne der vorliegenden Erfindung ist jede nicht-flüchtige (speicherbare) Information. Dabei kann es sich um eine Text-Mitteilung oder eine Sprach-Mitteilung oder ein anderes Format handeln. Vorzugsweise handelt es sich um eine Text-Mitteilung. Eine Sprach-Mitteilung kann mit bekannten Mitteln in eine Text-Mitteilung umgewandelt werden (siehe z.B. A. Clark et al.: The Handbook of Computational Linguistics and Natural Language Processing, Blackwell Handbooks in Linguistics, John Wiley & Sons, 2013, ISBN 9781118448670).

Eine Mitteilung im Sinne der vorliegenden Erfindung umfasst mindestens eine Information über einen Gesundheitszustand einer Person und/oder über eine Änderung eines Gesundheitszustands einer Person und/oder über eine Wirkung eines Arzneimittels und/oder über ein Ausbleiben einer Wirkung des Arzneimittels und/oder über eine Wirkung oder Nicht-Wirkung einer Therapie.

Beispiele für Mitteilungen sind
- Anfragen und Nebenwirkungsmeldungen von Patienten an pharmazeutische Unternehmen, Ärzte oder Apotheker zu pharmazeutischen Produkten
- Nebenwirkungsberichte von Ärzten, Pharmazeuten, Patienten, Lizenzpartnern, Gesundheitsbehörden
- wissenschaftliche Publikationen
- Ergebnisberichte zu klinischen Studien
- Informationen aus Patientenakten
- Mitteilungen von Nutzern in den so genannten sozialen Medien
- weitere Daten verschiedener Herkunft (z.B. Daten aus der Forschung und/oder Entwicklung von Arzneimitteln, der Sicherheitsüberwachung nach Zulassung eines Arzneimittels, der pharmazeutischen Industrie, von Gesundheitsbehörden (z.B. Behördensicherheitsdatenbanken), von universitären Einrichtungen oder anderen Bereichen (z.B. von Versicherungsunternehmen)

Vorzugsweise liegt die Mitteilung in digitaler Form vor. Der Begriff "digital" bedeutet, dass die Mitteilung von einer Maschine, in der Regel einem Computersystem, verarbeitet werden kann. Unter "Verarbeitung" werden die bekannten Verfahren zur elektronischen Datenverarbeitung (EDV) verstanden. Beispiele für Text-Mitteilungen in digitaler Form sind ASCII-Dateien oder XML-Dateien.

Liegt die Mitteilung in Form eines Druckerzeugnisses (z.B. als gedruckte Schrift in einem Dokument) vor, kann sie durch Einscannen z.B. mittels eines Flachbettscanners oder durch Abfotografieren (vorzugsweise mit einer Digitalkamera) und anschließende Texterkennung (*Optical Character Recognition*) in eine digitale Textform überführt werden.

Die Mitteilung kann in jeder Sprache formuliert sein. In einer Ausführungsform der vorliegenden Erfindung wird jede Mitteilung, die nicht auf Englisch formuliert ist, ins Englische übersetzt. Die Übersetzung kann Wort für Wort erfolgen. Die Übersetzung erfolgt vorzugsweise automatisiert mit einem maschinellen Übersetzungswerkzeug wie DeepL Translator, Google Translate, Microsoft Translate, Yandex.Translate, Amazon Translate, IBM Watson Language Translator, Babylon und/oder dergleichen.

Üblicherweise wird mindestens eine Mitteilung in digitaler Textform aus einer Datenbank, die ein Bestandteil des erfindungsgemäßen Computersystems sein kann oder mit diesem z.B. über eine Netzwerkverbindung verbunden sein kann, ausgelesen und in einen Arbeitsspeicher des erfindungsgemäßen Computersystems geladen.

Die Übertragung einer Mitteilung von einem separaten Computersystem auf das erfindungsgemäße Computersystem kann auf unterschiedlichem Wege erfolgen, z.B. über ein standardisiertes Übertragungsverfahren (z.B. ICH E2B), eine Schnittstelle zur Programmierung von Anwendungen (engl.: *application programming interface,* API), durch Laden der Mitteilung in eine Empfangseinheit des Computersystems usw.

Die in den Arbeitsspeicher des Computersystems geladene Mitteilung kann einem oder mehreren Vorverarbeitungsschritten unterzogen werden. Typische Vorverarbeitungsschritte sind Akronymauflösung, Tokenisierung, Lemmatisierung, Part-of-speech-Tagging und/oder Datenbereinigung.

Bei der Akronymauflösung wird ein Akronym durch seine erweiterte Form ersetzt. Zu diesem Zweck kann ein Wörterbuch mit Akronymen verwendet werden. Vorzugsweise wird ein Wörterbuch für ein oder mehrere spezifische Arzneimittel und/oder medizinische Krankheitsbilder erstellt und gepflegt, insbesondere von solchen Erkrankungen, die von dem einen oder den mehreren Arzneimitteln therapiert werden. In einem solchen Wörterbuch sind Akronyme und ihre Bedeutungen/Erweiterungen aufgeführt, die häufig im Zusammenhang mit den spezifischen Arzneimitteln und Erkrankungen verwendet werden.

Der Begriff "Wörterbuch" bezieht sich in dieser Beschreibung vorzugsweise auf eine oder mehrere elektronische Datenbanken (z.B. relationale Datenbanken) oder andere Datenstrukturen (z.B. Python DataFrames), in der / in denen die entsprechenden Informationen digital gespeichert sind und von dort abgerufen werden können.

Die Tokenisierung ist ein grundlegender NLP-Prozess (NLP: *Natural Language Processing*)*.* Bei der Tokenisierung wird ein Text in kleinere Einheiten wie einzelne Wörter oder Begriffe aufgeteilt. Jede dieser kleineren Einheiten wird als Token bezeichnet. Details zur Tokenisierung sind in der Literatur zu finden (siehe z.B. Handbook of Natural Language Processing, Chapman & Hall/CRC: Machine Learning & Pattern Recognition, ISBN-13: 978-1420085921; J. J. Webster et al.: Tokenization as the initial phase in NLP, Proc. of COLING-92, NANTES, AUG. 23 28, 1992; WO2010/132790A1).

Für die Tokenisierung einer Mitteilung stehen verschiedene Werkzeuge zur Verfügung, mit denen die Mitteilung automatisiert vorverarbeitet werden kann; ein Beispiel für ein solches Werkzeug ist scispaCy (siehe z. B. M. Neumann et al.: ScispaCy: Fast and Robust Models for Biomedical Natural Language Processing, Proceedings ofthe BioNLP, 2019, Seiten 319-327, arXiv:1902.07669).

Die Lemmatisierung ist ein Verfahren zur Bestimmung des Lemmas eines Wortes anhand seiner beabsichtigten Bedeutung. In vielen Sprachen erscheinen Wörter in verschiedenen Flexionsformen. Im Englischen kann das Verb *"to walk"* beispielsweise als *"walk", "walked", "walks"* oder *"walking"* erscheinen. Die Grundform *"walk",* die man in einem Wörterbuch nachschlagen kann, wird als Lemma für das Wort bezeichnet. Der Prozess der Lemmatisierung ist also die Substitution von Wörtern oder Phrasen in gebeugten Formen durch ihr Lemma. Details zur Lemmatisierung können in der Literatur gefunden werden (siehe z.B. Liu et al. Journal of Biomedical Semantics 2012, 3:3; EP3248111A1; https://doi.org/10.26615/978-954-452-049-6_006).

Unter Part-of-speech-Tagging (POS-Tagging) versteht man die Zuordnung von Wörtern und Satzzeichen einer Mitteilung zu Wortarten. Diese Methode dient der Analyse der Satzstruktur. Es existieren verschiedene POS-Tagger (z.B. Penn Treebank der Pennsylvania University https://catalog.ldc.upenn.edu/LDC99T42, Stanford Log-linear Part-Of-Speech Tagger https://nlp.stanford.edu/software/tagger.shtml, NLTK POS-Tagging: www.nltk.org, (siehe z.B.: M. Neunerdt et al.: Part-Of-Speech Tagging for Social Media Texts, 2013, DOI: 10.13140/2.1.2905.7284))

Die Datenbereinigung (engl.: *data cleansing*) ist ein Verfahren zur Verbesserung der Informationsqualität (z.B. durch Entfernung von Duplikaten, störenden und nicht erforderlichen Daten, z.B. je nach Anwendungsfall die Entfernung von Emojis).

Beim Entfernen nicht informativer Phrasen und / oder Wörter werden Wörter, die einem Text keine Bedeutung verleihen, aber häufig vorkommen, entfernt. Diese Wörter werden auch als Stoppwörter bezeichnet. Es gibt einige Kataloge mit Stoppwörtern (siehe z. B. https://www.textfixer.com/tutorials/common-english-words.txt, https://dev.mysql.com/doc/refman/8.0/en/, fulltext-stopwords.html, http://xpo6.com/list-of-englishstop-words/). Techniken zum Entfernen von Stoppwörtern und nicht informativen Phrasen können in diversen Publikationen gefunden werden (siehe z.B. EP3232336A1, US20060224572, US20080204595, WO2017/048584).

Tippfehler können durch Einsatz von Methoden aus der Ähnlichkeitsanalyse identifiziert werden (z. B. Jaro-Winkler string metric: Matthew A. Jaro (1989) Advances in Record-Linkage Methodology as Applied to Matching the 1985 Census of Tampa, Florida, Journal of the American Statistical Association, 84:406, 414-420, DOI: 10.1080/01621459.1989.10478785).

Es existieren Werkzeuge, die eine Vorverarbeitung von Mitteilungen automatisiert vornehmen, als Beispiel sei das NLP-Werkzeug namens *Stanford CoreNLP* genannt (siehe: https://stanfordnlp.github.io/CoreNLP/).

Die empfangene Mitteilung wird (ggf. nach Vorverarbeitung) in einem weiteren Schritt einer Sentiment-Analyse (Stimmungserkennung) unterzogen.

In diesem Verfahrensschritt wird analysiert, ob es sich bei der empfangenen Mitteilung um eine positive Mitteilung handelt. Dieser Schritt erfolgt vorzugsweise in Form einer Klassifikation der Mitteilung. Dabei wird die Mitteilung einer von mehreren Klassen zugeordnet. Die Zahl der Klassen kann beispielsweise 2, 3, 4, 5 oder mehr betragen. In einer bevorzugten Ausführungsform erfolgt die Klassifizierung in eine von zwei Klassen (z.B. positiv und nicht-positiv). Es ist aber auch denkbar, dass die Klassifizierung in eine von drei Klassen (z.B. positiv, neutral, negativ), in eine von vier Klassen (z.B. positiv, eher positiv, eher negativ, negativ) oder in eine von fünf Klassen (z.B. sehr positiv, positiv, neutral, negativ, sehr negativ) erfolgt. Weitere Möglichkeiten sind denkbar.

Eine "positive Mitteilung" im Sinne der vorliegenden Erfindung ist eine Mitteilung, die eine Aussage über eine Verbesserung eines Gesundheitszustands eines Patienten enthält. Eine "negative Mitteilung" ist dementsprechend eine Mitteilung, die eine Aussage über eine Verschlechterung eines Gesundheitszustands eines Patienten oder über einen unveränderten Gesundheitszustand eines Patienten enthält, obwohl der Autor der Mitteilung eine Verbesserung durch die Einnahme des Arzneimittels erwartet hätte (das entspräche z.B. einem der MedDRA Preferred Terms (PTs) *"drug ineffective", "therapeutic response decreased"* oder einem ähnlichen Term). Eine "positive Mitteilung" im Sinne der vorliegenden Erfindung kann auch eine Aussage über ein Arzneimittel/Medikament/Wirkstoff enthalten, wobei dem Arzneimittel/Medikament/Wirkstoff in der Aussage eine positive (gesundheitsfördernde) Wirkung zugesprochen wird; während eine "negative Mitteilung" eine Aussage über ein Arzneimittel/Medikament/Wirkstoff umfasst, die dem Arzneimittel/Medikament/Wirkstoff eine negative (gesundheitsabträgliche) Wirkung zuspricht.

Es erfolgt also eine Zuordnung der Mitteilung in eine von mindestens zwei Klassen, wobei die mindestens zwei Klassen eine erste Klasse (mit positiven Mitteilungen) umfassen, wobei die Mitteilung in die erste Klasse eingeordnet wird, wenn die Mitteilung eine Aussage über eine Verbesserung des Gesundheitszustands der Person enthält und/oder wenn die Mitteilung eine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird.

Liegen zwei Klassen vor, können in die zweite Klasse (mit nicht-positiven Mitteilungen) diejenigen Mitteilungen eingeordnet werden, die keine Aussage über eine Verbesserung des Gesundheitszustands der Person enthält und/oder keine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird.

Liegen drei Klassen vor, können in die zweite Klasse (mit negativen Mitteilungen) diejenigen Mitteilungen eingeordnet werden, die eine Aussage über eine Verschlechterung des Gesundheitszustands einer Person enthält und/oder eine Aussage über ein Arzneimittel und/oder eine Therapie enthält, in der dem Arzneimittel und/oder der Therapie eine gesundheitsabträgliche und/oder nicht-symptomlindernde oder eine ausbleibende Wirkung zugesprochen wird. In die dritte Klasse (mit neutralen Mitteilungen) können diejenigen Mitteilungen eingeordnet werden, die nicht der ersten und nicht der zweiten Klassen zugeordnet werden können.

Weitere Möglichkeiten sind denkbar.

Eine Möglichkeit für die Klassifizierung einer Mitteilung besteht in der Analyse einzelner Bestandteile der Mitteilung (wie z.B. Sätze, Satzbestandteile, Token, Phrasen und/oder Wörter) und der Bewertung, ob die Bestandteile eine (überwiegend) positive Konnotation oder eine (überwiegend) negative Konnotation haben.

Dies kann beginnend auf Basis der Wörter mit Hilfe eines Wörterbuchs erfolgen, in dem für einzelne Wörter ein Attribut verzeichnet ist, das angibt, ob es sich bei dem jeweiligen Wort um ein positives oder negatives Wort handelt.

Ein positives Attribut können dabei beispielsweise Wörter wie "helfen", "verbessern", "gut" erhalten, während Wörter wie beispielsweise "verschlechtern", "schlecht" ein negatives Attribut erhalten können.

Falls in dem zu untersuchenden Text Krankheiten und/oder Nebenwirkungen beschrieben werden, wird dem entsprechenden Wort vorzugsweise ein negatives Attribut zugeordnet.

Diese Wörter, die einen erheblichen Einfluss auf eine positive oder negative Attribuierung besitzen, werden in dieser Beschreibung auch als "effektive Terme" bezeichnet und sind idealerweise in einem Wörterbuch hinterlegt und können automatisiert abgerufen werden.

Dabei können Negationen wie beispielsweise die Wörter "nicht" und "kein" einem Wort, das vormals positiv oder neutral eingeordnet wurde, ein negatives Attribut verleihen.

Zur Identifizierung und Attribuierung medizinischer Fachausdrücke kann beispielsweise jedes einzelne Wort der Mitteilung mit einem (digitalen) Wörterbuch, in dem Krankheiten verzeichnet sind, abgeglichen werden. Dieses Wörterbuch enthält neben der medizinischen Terminologie idealerweise auch umgangssprachliche Ausdrücke. Als Basis kann ein medizinisches Verzeichnis verwendet werden (z. B. MedDRA www.meddra.org), das mit Synonymen erweitert wurde, um gerade im Bereich der sozialen Medien Ausdrücke wie *"I had a face like a lobster"* einem medizinischen Terminus (z.B. dem MedDRA Preferred Term (PT) *"rash"*) zuordnen zu können. Tippfehler können wiederum mit Hilfe der Ähnlichkeitsanalyse identifiziert werden.

Vorzugsweise erhalten alle Worte initial ein neutrales Attribut (Sentiment) und die Identifizierung und Klassifizierung der einzelnen Worte mit positiven, negativen und/oder neutralen Attributen erfolgt mit Hilfe eines Wörterbuchs, idealerweise durch vorherige Lemmatisierung der zu prüfenden Tokens und der Wörterbucheinträge. Positive, negative und ggf. neutrale Attribute werden in eine maschinenlesbare Form übertragen, zum Beispiel durch Verwendung von Kennnummern (z.B. 1: negativ, 2: neutral, 3: positiv). Eine Verfeinerung ist ebenfalls möglich (z.B. 0: sehr negativ, 1: negativ, 2: neutral, 3: positiv, 4: sehr positiv). Es ist denkbar, dass vor der Attribuierung zunächst die Wortarten für die Wörter einer Mitteilung (Nomen, Verb, Adjektiv, Adverb, Zahlwort usw.) ermittelt werden. Es ist denkbar, dass einige Wortarten bei der Klassifizierung höher gewichtet werden als andere (z. B. Verben höher als Nomen). Es kann auch neutrale Attribute geben, d.h. das Wort selbst gibt der Aussage weder eine positive noch eine negative Konnotation.

Zur Auswertung von Mitteilungen aus den sozialen Medien können insbesondere eine Identifizierung und Attribuierung von Emojis sinnvoll sein.

Ob eine Mitteilung als positiv oder negativ (oder neutral) klassifiziert wird, kann beispielsweise durch Addieren der Anzahlen der positiven und der negativen Attribute der einzelnen Wörter (oder Token oder Phrasen) erfolgen. Je nachdem, ob die positiven oder negativen Attribute überwiegen, kann die Mitteilung als positiv oder negativ klassifiziert werden. Halten sich positive und negative Attribute die Waage oder finden sich keine positiven oder negativen Attribute in einer Mitteilung, kann die Mitteilung als neutral klassifiziert werden. Denkbar ist auch, dass eine Mitteilung bereits dann als positiv klassifiziert wird, wenn sie mindestens ein positives Attribut aufweist (unabhängig von der Zahl der negativen Attribute). Denkbar ist auch, dass die Zahl der positiven Attribute (absolut oder im Verhältnis zur Zahl der negativen Attribute) einen vordefinierten Schwellenwert überschreiten muss, damit eine Mitteilung insgesamt als positiv eingestuft wird.

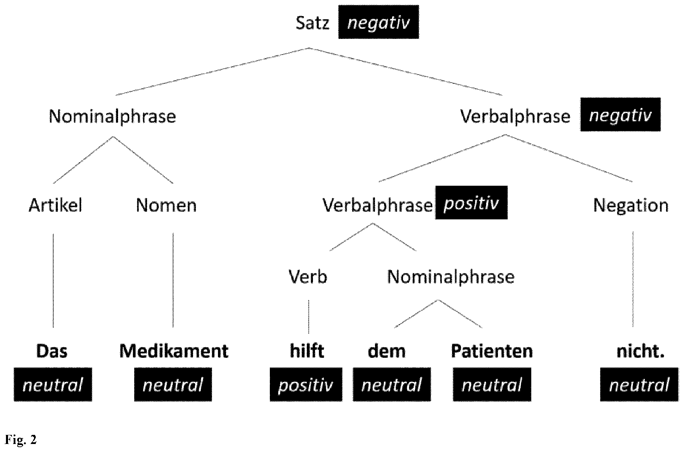

In einer bevorzugten Ausführungsform wird die in NLP-Werkzeugen vorgegebene Identifizierung der Satzstruktur für die Attribuierung der Satzbestandteile und die Klassifizierung der Mitteilung verwendet. Fig. 2 zeigt eine solche Attribuierung und Klassifizierung in Form eines Beispiels.

NLP-Werkzeuge gehen üblicherweise bei der Identifizierung der Satzstruktur von vollständigen, korrekten Sätzen in der jeweiligen Sprache aus. Gerade in den sozialen Medien werden jedoch unvollständige Sätze (z.B. unter Weglassung des Subjektes, sog. Ellipsis) gepostet. In einem solchen Fall kann der Satz durch NLP-Werkzeuge automatisiert in eine syntaktisch korrekte Form gebracht oder die Satzstrukturanalyse des NLP-Werkzeugs für die Weiterverarbeitung des unvollständigen Satzes verwendet werden. In einer bevorzugten Ausführungsform wird die potenziell fehlerhafte Satzstrukturanalyse des NLP-Werkzeugs verwendet. Weitere Möglichkeiten sind denkbar.

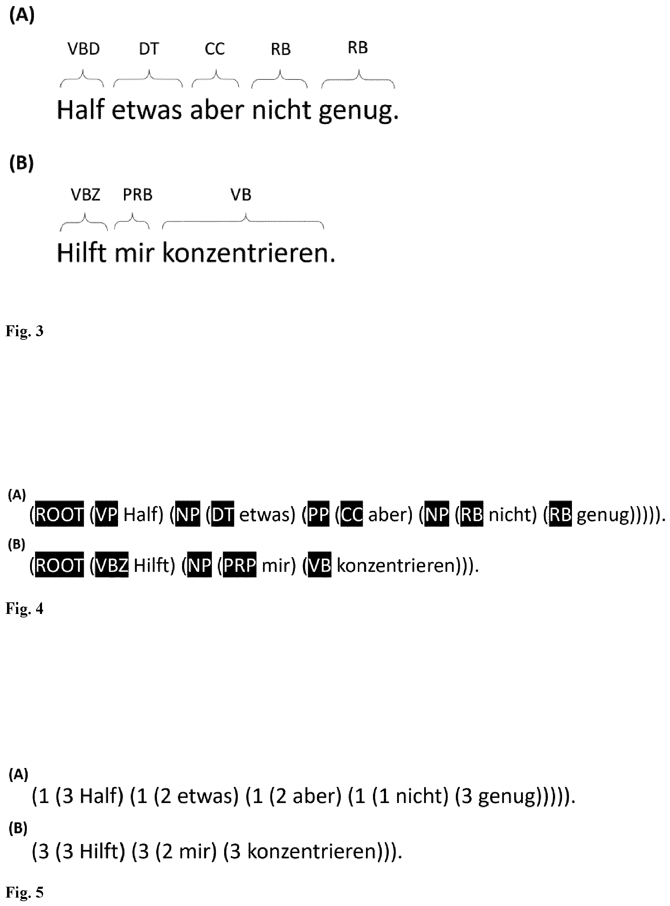

Fig. 3 zeigt zwei Beispiele für syntaktisch unkorrekte und unvollständige Sätze. Der Satz (A) kann insgesamt als negativ, der Satz (B) insgesamt als positiv eingestuft werden. Für das POS-Tagging wurden die von der Penn Treebank veröffentlichten Tags verwendet (siehe z.B. https://www.ling.upenn.edu/courses/Fall_2003/ling001/ penn_treebank_pos.html). Dabei bedeuten VBD: *verb, past tense* (Verb, Vergangenheitsform), DT: *determiner* (Bestimmungswort), CC: *coordinating conjunction* (Konjunktion), RB: *adverb* (Adverb), VBZ: *verb, 3^{rd} person singular present* (Verb, dritte Person singular Präsenz), PRP: *personal pronoun* (Personalpronomen), VB: *verb, base form* (Verb, Grundform).

Fig. 4 zeigt die unvollständigen Sätze aus Fig. 3 und das Ergebnis einer Satzstrukturanalyse durch ein anderes NLP-Werkzeug. Dieses NLP-Werkzeug analysiert die Satzstruktur und bildet Klammern um zusammengehörige Ausdrücke, die in Verbalphrase (VP) und Nominalphrase (NP) und andere linguistische Satzstrukturen unterteilt werden können (z. B. Adverb-Phrase).

Fig. 5 zeigt beispielhaft das Ergebnis eines Attribuierungsprozesses für die in Fig. 4 aufgeführten unvollständigen Sätze (A) und (B). Zunächst wurden die POS-Tags aus Fig. 4 durch positive, neutrale und negative Attribute ersetzt (im vorliegenden Fall 1: negativ, 2: neutral, 3: positiv). Danach erfolgte die Attribuierung der Satzstrukturelemente (in unserem Beispiel ROOT, NP, VP). Hierbei erfolgte die Attribuierung vorzugsweise entsprechend den Klammern und der bereits attribuierten POS-Tags. Eine Negation (in unserem Beispiel "nicht") verändert ein positives Attribut in ein negatives Attribut und umgekehrt, d.h. aus (NP(1 nicht) (3 genug)) wird (1(1 nicht)(3 genug)). Die Ersetzung des Tags "ROOT" charakterisiert, ob der gesamte Ausdruck ein positives, neutrales oder negatives Attribut trägt.

In einer weiteren Ausführungsform der vorliegenden Erfindung wird zur Klassifizierung der Mitteilung ein Modell des maschinellen Lernens (engl.: *machine learning model*) verwendet, das z.B. in einem überwachten Lernverfahren (engl. *supervised training*) oder in einem unüberwachten Lernverfahren (engl. *unsupervised training*) trainiert worden ist, ein empfangene Mitteilung in eine von mehreren Klassen einzuordnen, z.B. die Mitteilung als positiv oder negativ (oder neutral) zu klassifizieren (Klassifikationsmodell).

Es ist denkbar, dass ein bereits vortrainiertes Modell verwendet wird, das durch das Vortrainieren bereits semantisches Sprachwissen beinhaltet. Als ein solches vortrainiertes Modell eignet sich beispielsweise das Model BERTweet, ein auf Basis englischer Tweets vortrainiertes Modell (siehe z.B.: D. Q. Nguyen et al.: BERTweet: A pre-trained language model for English Tweets, arXiv:2005.10200), oder ein Modell, das mit Hilfe naturwissenschaftlicher oder medizinischer Texte vortrainiert worden ist, wie ScispaCy (siehe z.B.: M. Neumann et al., ScispaCy: Fast and Robust Models for Biomedical Natural Language Processing, arXiv: 1902.07669, 2019), SciBERT (siehe z.B.: I. Beltagy et al.: SciBERT: Pretrained Language Model for Scientific Text, arXiv: 1903.1067) oder BioBERT (siehe z.B.: J. Lee et al.: BioBERT: a pre-trained biomedical language representation model for biomedical text mining, Bioinformatics 2020, pages 1234-1240).

Das (vortrainierte) Modell wird als Klassifikator (engl.: *classifier*) konfiguriert und mit Hilfe annotierter Mitteilungen (d.h. anhand von Mitteilungen, die bereits (z.B. von einem Menschen) als positiv oder negativ (oder neutral) eingestuft worden sind) trainiert (*fine-tuning*). Der trainierte Klassifikator kann dann verwendet werden, neue (unbekannte) Mitteilungen zu klassifizieren.

Verfahren zur Klassifikation von Daten sind vielfältig in der Literatur beschrieben. Bei dem Klassifikationsmodell kann es sich um beispielsweise um ein künstliches neuronales Netz, um ein Naive Bayes Model, stochastisches Gradientenabstiegsmodell, ein k-nächste-Nachbarn-Modell, einen Entscheidungsbaum, ein Random Forest Model, ein Support Vector Machine Model, um ein Gradient Boosting Model oder um ein anderes Modell handeln (siehe z.B.: Charles Bouveyron et al.: Model-Based Clustering and Classification for Data Science: With Applications in R, Vol. 50, Cambridge Series in Statistical and Probabilistic Mathematics, 2019, ISBN 9781108640596).

Es ist denkbar, das Modell des maschinellen Lernens mit einem Trainings-Datensatz zu trainieren, der mittels der oben beschriebenen Satzstrukturanalyse und Attribuierung der Satzelemente gewonnen worden ist.

Eine Möglichkeit, den Trainingsprozess zu automatisieren, besteht in der Verwendung von Nebenwirkungs-, Produkt- oder Indikationsinformationen aus einer Pharmakovigilanz-Sicherheitsdatenbank.

Solche Nebenwirkungs-, Produkt- oder Indikationsinformationen sind in dieser Datenbank in der Regel manuell editiert und bewertet. Positive Wirkungen eines Arzneimittels sind ebenfalls als Nebenwirkungsmeldungen erfasst und können somit als Datensatz für ein automatisiertes Training verwendet werden.

Dabei kann der Originaltext der Mitteilung (sog. *reported term*) und die zugehörende kodierte Information (sog. *coded term*) verwendet werden. Die kodierte Information ist einem standardisierten medizinischen Term (MedDRA PT) zugeordnet. Für positive Arzneimittelwirkungen können zum Beispiel folgende MedDRA Preferred Terms als "True Positive" verwendet werden: *"pre-existing condition improved"* (bereits bestehender Zustand verbessert), *"therapeutic response unexpected"* (therapeutische Reaktion unerwartet), *"drug effective for unapproved indication"* (Medikament wirksam für nicht genehmigte Indikation).

Für negative Arzneimittelwirkungen können zum Beispiel Nebenwirkungsmeldungen mit folgenden MedDRA PTs verwendet werden: *"therapeutic response decreased"* (therapeutisches Ansprechen nahm ab), *"therapeutic product ineffective for unapproved indication"* (therapeutisches Produkt unwirksam für nicht genehmigte Indikation), *"drug ineffective for unapproved indication"* (Medikament unwirksam für nicht genehmigte Indikation), *"therapeutic product ineffective"* (therapeutisches Produkt unwirksam), *"device ineffective"* (Gerät unwirksam), *"drug ineffective"* (Wirkstoff unwirksam).

Es ist auch denkbar, neutrale Aussagen zusätzlich zu den positiven und negativen Aussagen zu trainieren. Falls eine neutrale Aussage mittrainiert werden soll, können z.B. MedDRA PTs, die nicht als positiv oder negativ zugeordnet sind, als neutral eingestuft und im Training verwendet werden.

Ein automatisiertes Training erlaubt eine deutliche und schnelle Erweiterung des Datensatzes.

Bei der Erstellung eines Trainingsdatensatzes ist es von Vorteil, wenn die Anzahlen der positiven, negativen, und ggf. neutralen Datensätze gleich oder annähernd gleich sind.

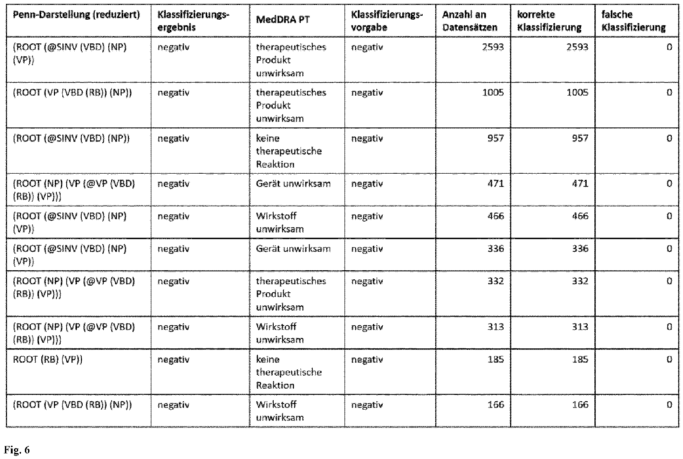

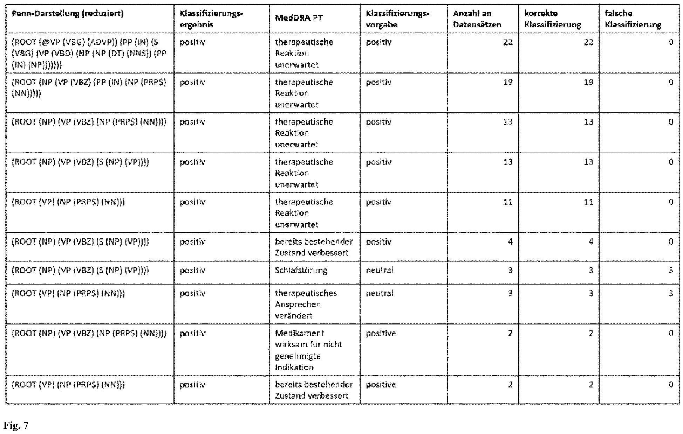

Fig. 6 und Fig. 7 zeigen beispielhaft die Ergebnisse einer automatisierten Klassifizierung von Nebenwirkungsmeldungen aus einer Pharmakovigilanz-Sicherheitsdatenbank.

Zusätzlich zur Sentiment-Analyse können weitere Verfahren eingesetzt werden, um die korrekte Klassifizierung von Mitteilungen zu verbessern, und neben der positiven Wirkung auch andere wichtige medizinische Information in der Mitteilung zu identifizieren.

In einer bevorzugten Ausführungsform wird in der Mitteilung ein Arzneimittel identifiziert, dem die positive, negative oder ggf. neutrale Wirkung zugesprochen wird. Dazu kann beispielsweise jedes einzelne Wort der Mitteilung mit einem (digitalen) Wörterbuch, in dem Arzneimittel verzeichnet sind, abgeglichen werden. Kommt es dabei zu einer Übereinstimmung, dann ist das entsprechende Arzneimittel identifiziert. Vorzugsweise sind in dem Wörterbuch nicht nur Arzneimittel, sondern auch Medikamente, Handelsnamen, Namen von Wirkstoffen, Internationale Freinamen (engl.: *international non-proprietary names,* INNs) und dergleichen gespeichert. So können für den Wirkstoff Acetylsalicylsäure auch der IUPAC-Name (2-Acetoxybenzoic acid), ein Markenname eines Medikaments (z.B. Aspirin), eine gebräuchliche Abkürzung (z.B. ASS) und/oder sonstige Kennungen als zusätzliche Identifikatoren gespeichert sein. Neben einer regelbasierten Identifizierung wird auch eine Identifizierung mit Hilfe von NLP Systemen eingesetzt, die Verwendung von Spitznamen (z.B. *Zolpi* anstatt *Zolpidem*)*,* Mehrdeutigkeiten von Handelsnamen (z.B. Yasmin, Intermezzo) mit identischen Begriffen aus anderen Bereichen auflöst.

In einer bevorzugten Ausführungsform wird in einem weiteren Schritt in der Mitteilung eine Indikation identifiziert.

Behördlich zugelassene Arzneimittel / Medikamente werden üblicherweise für eine definierte Indikation zugelassen; neue Arzneimittel / Medikamente werden üblicherweise für eine oder mehrere Indikationen entwickelt.

Als "Indikation" bezeichnet man den Grund für den Einsatz einer therapeutischen oder diagnostischen Maßnahme bzw. welche medizinische Maßnahme bei einem bestimmten Krankheitsbild angebracht ist. Bei Arzneimitteln spricht man in diesem Zusammenhang auch vom Anwendungsgebiet oder einer Anwendung.

Arzneimittel und Medikamente werden aber auch für nicht zugelassene Indikationen eingesetzt (*off-label use*)*.* Eine solche nicht-zugelassene Verwendung kann einen Hinweis auf neue unbekannte Indikationen geben.

In einem Großteil der Mitteilungen, die erfindungsgemäß analysiert werden, insbesondere der Mitteilungen, die von Patienten stammen, werden Indikationen selbst nicht namentlich benannt, sondern es werden stattdessen eher Symptome aufgeführt. Es gilt also, aus den Mitteilungen diejenigen Indikationen, die in den Mitteilungen üblicherweise indirekt, selten direkt angesprochen werden, zu identifizieren. Dies geschieht vorzugsweise wieder unter Verwendung eines vortrainierten Modells wie beispielsweise BERTweet, ScispaCy, SciBERT, BioBERT oder einem anderen Modell.

Das vortrainierte Modell kann wiederum als ein Klassifikator konfiguriert und anhand von annotierten Mitteilungen (d.h. Mitteilungen, in denen eine oder mehrere angesprochene Indikationen z.B. durch einen Menschen bereits identifiziert worden sind) trainiert (*fine-tuning*), einen Zusammenhang zwischen Mitteilungen und den darin angesprochenen Indikationen zu lernen. Der trainierte Klassifikator kann dann verwendet werden, neue (unbekannte) Mitteilungen einer Indikation (oder mehreren Indikationen) zuzuordnen.

In einem weiteren Schritt kann analysiert werden, ob die mindestens eine, in der Mitteilung identifizierte Indikation bereits eine, für das in der Mitteilung identifizierte Arzneimittel bekannte Indikation ist. Hierzu wird vorzugsweise ein Wörterbuch herangezogen, in dem für mindestens ein Arzneimittel (vorzugsweise für eine Vielzahl an Arzneimitteln) verzeichnet ist, für welche Indikation(en) das mindestens eine Arzneimittel zugelassen ist und/oder entwickelt wird.

Wird die in der Mitteilung identifizierte Indikation mit den Indikationen in dem Wörterbuch verglichen, die dort für das in der Mitteilung identifizierte Arzneimittel aufgeführt sind, und kommt es zu einer Übereinstimmung, handelt es sich bei der identifizierten Indikation um eine bereits für das identifizierte Arzneimittel bekannte Indikation. Kommt es hingegen zu keiner Übereinstimmung, ist es denkbar, dass für das Arzneimittel eine neue Anwendung (eine neue Indikation) gefunden worden ist.

Eine Unterscheidung zwischen einer positiven Arzneimittelwirkung bei einer zugelassenen Indikation und einer positiven Arzneimittelwirkung, die auf eine neue Indikation hindeutet, ist also potenziell möglich. Es kann jedoch nicht immer vorausgesetzt werden, dass der Zulassungsstatus eines Arzneimittels dem Autor der Mitteilung bekannt ist, d.h. ob die in der Mitteilung aufgeführte positive Arzneimittelwirkung nicht tatsächlich bereits zugelassen ist. Andererseits kann es sich bei einer semantisch identifizierten Indikation auch um eine missbräuchliche Anwendung eines Arzneimittels oder um einen medizinisch verordneten "Off-Label Use" handeln. Daher werden vorzugsweise alle Mitteilungen, die eine positive Arzneimittelwirkung beinhalten, weiter untersucht.

Um eine wissenschaftliche Auswertung einer identifizierten positiven Arzneimittelwirkung zu unterstützen, kann ein Abgleich bekannter positiver Arzneimittelwirkungen des Arzneimittelwirkstoffs (gemeint ist die pharmakologisch aktive Substanz) unabhängig von Handelsnamen und Arzneimitteln einzelner pharmazeutischer Hersteller auf Substanzklassen von Arzneimitteln (z. B. Protease-Inhibitoren) erweitert werden.

In einer besonders bevorzugten Ausführungsform wird die Mitteilung einem Klassifikationsmodell zugeführt, das für die Mitteilung eine oder mehrere Wahrscheinlichkeiten dafür ausgibt, dass die Mitteilung ein Arzneimittel, eine positive Arzneimittelwirkung, eine Indikation, eine Begleiterkrankung, eine Aussage über einen (un)beabsichtigten Effekt und/oder ähnliches enthält. Vorzugsweise wird eine sogenannte Pipeline eingerichtet (https://huggingface.co/transformers/main_classes/pipelines.html), die eine Vorsortierung der Mitteilungsinhalte ermöglicht.

Anschließend kann ein so genannter "Span" bestimmt werden. Hierbei erfolgt eine Identifizierung, an welcher/n Wortposition(en) ein Arzneimittel, eine positive Arzneimittelwirkung usw. erwähnt sind. Ein übliches Span-Klassifizierungsverfahren ist z.B. das sogenannte BIO-Tagging (B: *Beginning,* I: *Inside,* O: *Outside*)*.* Hierbei werden die einzelnen Token einer Mitteilung einem Merkmal zugeordnet (z.B. positive Arzneimittelwirkung). Dieses kann für die einzelnen Merkmale separat oder gemeinsam als Multiklassifikator erfolgen.

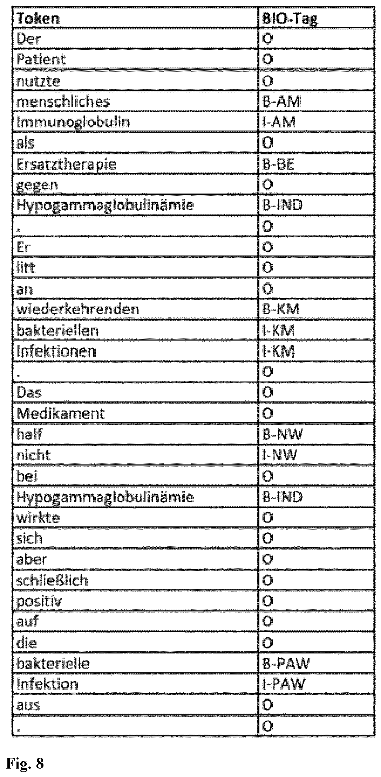

Fig. 8 zeigt ein Beispiel für ein BIO-Tagging. In dem Beispiel wurde folgende Mitteilung analysiert:
*DerPatient nutzte menschliches Immunglobulin als Ersatztherapie gegen Hypogammaglobulinämie. Er litt an wiederkehrenden bakteriellen Infektionen. Das Medikament half nicht bei Hypogammaglobulinämie wirkte sich aber schließlich positiv auf die bakterielle Infektion aus.*

Für das BIO-Tagging wurden die folgenden Merkmale verwendet: NW: Nebenwirkung, PAW: Positive Arzneimittelwirkung, AM: Arzneimittel, IND: Indikation, BE: Beabsichtigter Effekt, KM: Begleiterkrankung/Komorbidität. Komorbidität oder "Begleiterkrankung" ist ein weiteres, diagnostisch identifizierbares Krankheitsbild und bedeutet, dass zusammen mit einer Grunderkrankung (Indexerkrankung) gleichzeitig eine oder mehrere weitere Krankheiten vorliegen. Der beabsichtigte Effekt ("*intended use"*) beschreibt die beabsichtigte Wirkung, Ziel oder Strategie, die durch das Arzneimittel erzielt werden soll. Beispiele sind Prävention, Prophylaxe, Diagnose, Palliativtherapie, Behandlung, passive Immunisierung usw.

Das Span-Klassifizierungsverfahren beinhaltet also nicht nur eine Klassifizierung, sondern auch eine Identifizierung der Textstellen, die auf einen positiven Effekt hinweisen.

Wie beschrieben gilt es, positive Arzneimittelwirkungen von anderen Arzneimittelinformationen (zum Beispiel Begleiterkrankungen oder Nebenwirkungsmeldungen) zu separieren. Dazu können Modelle entwickelt werden, die Indikationsinformationen, Begleiterkrankungen, Nebenwirkungsmeldungen, und beabsichtigte Effekte in Mitteilungen identifizieren. Diese können als separate Modelle entwickelt und trainiert werden. Denkbar sind auch die Entwicklung und das Trainieren eines einheitlichen Modells zur Erkennung aller genannten Merkmale.

Die Ergebnisse der Analyse der empfangenen Mitteilung (oder einer Vielzahl an empfangenen Mitteilungen) kann gegenüber einem Nutzer ausgeben (z.B. auf einem Bildschirm angezeigt oder auf einem Drucker ausgedruckt), an ein (separates) Computersystem zur weiteren Analyse übermittelt und/oder in einem Datenspeicher gespeichert werden.

Ein solches Ergebnis kann eine Information enthalten, dass für ein Arzneimittel eine mögliche neue Anwendung identifiziert worden ist. Das Ergebnis kann eine Information über das jeweilige Arzneimittel und/oder die jeweilige, möglicherweise neue Indikation enthalten. Falls eine Zuordnung der Mitteilung zu einem Land möglich ist, in dem das Arzneimittel gekauft oder verwendet wurde, kann diese Information ebenfalls genutzt werden, da länderspezifische Zulassungen unterschiedliche Indikationen ermöglichen. Falls eine Zuordnung zu dem Land nicht möglich ist, können Zulassungsinformationen für ein Arzneimittel aller Länder zusammengefasst und als Grundlage für einen Vergleich herangezogen. Das Ergebnis kann auch Scoring-Werte (Wahrscheinlichkeiten) für die identifizierten Merkmale, eine Zuordnung der Worte zu den Merkmalen und/oder die ursprüngliche Mitteilung selbst enthalten, deren Analyse zu dem Ergebnis geführt hat. Das Ergebnis kann auch einen Link enthalten, über den man die ursprüngliche Mitteilung, deren Analyse zu dem Ergebnis geführt hat, abrufen kann.

In einem weiteren Schritt können Untersuchungen initiiert und ausgeführt werden, die eine Verifikation oder Falsifikation der Hypothese, es liegt für das Arzneimittel eine neue Indikation vor, erlauben.

Wie bereits beschrieben kann die vorliegende Erfindung dafür verwendet werden, neue Anwendungsmöglichkeiten für Arzneimittel zu identifizieren.

Die vorliegende Erfindung kann ferner dafür verwendet werden, Informationen zu Arzneimitteln aus verschiedenen Quellen automatisiert zu extrahieren und verschiedenen Kategorien zuzuordnen.

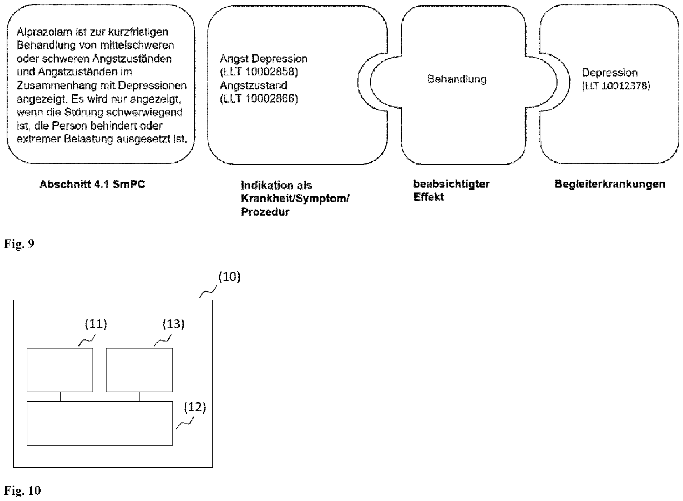

Die europäische Zulassungsbehörde für medizinische Produkte (EMA) sieht in einem Richtlinienentwurf *"*Product Management Services -Implementation of International Organization for Standardization (ISO) standards for the identification of medicinal products (IDMP) in Europe" vom Februar 2020 vor, dass zukünftig Arzneimittel entsprechend ISO IDMP zu spezifizieren sind (z.B. Begleiterkrankungen, beabsichtigter Effekt) (siehe (https://www.ema.europa.eu/en/documents/regulatory-procedural-guideline/product-managementservices-implementation-international-organization-standardization-iso-standards_en.pdf). Zurzeit liegen Informationen z.B. in Sektion 4.1 des SmPC vor und müssten manuell identifiziert und extrahiert werden. Ein Beispiel ist in Fig. 9 gezeigt.

Hier kann die vorliegende Erfindung eingesetzt werden, um eine automatisierte Datenextraktion und Zuordnung durchzuführen.

Das erfindungsgemäße Verfahren umfasst eine Reihe von Schritten, die sequenziell oder teilweise parallel zueinander ausgeführt werden können:
Das erfindungsgemäße Verfahren kann ferner in Form eines Filters durchgeführt werden. Dabei werden Verfahrensschritte ausgeführt und anhand des Ergebnisses der Verfahrensschritte entschieden, ob das Verfahren weiter ausgeführt, konditional prozessiert oder abgebrochen wird. Ein Beispiel hierfür ist der folgende Ablauf:
In einem ersten Schritt wird eine Mitteilung empfangen. Die Datenquelle wird identifiziert und entweder besitzen die Daten dieser Quelle voreingestellte Formate und Sprache, die Mitteilung enthält Sprach- und Formatinformationen als Metadaten (z. B. XML Tags) oder eine Identifizierung erfolgt nach Empfang. Anschließend wird ggf. eine Transformation in eine digitale Information in englischer Sprache vorgenommen.

In einem zweiten Schritt wird die Mitteilung, vorverarbeitet und geprüft, ob in der Mitteilung ein Arzneimittel identifiziert werden kann, und/oder es wird geprüft, ob in der Mitteilung eine Aussage über eine positive Wirkung eines Arzneimittels und/oder über eine Verbesserung eines Gesundheitszustands einer Person identifiziert werden kann. Dazu wird die Mitteilung idealerweise sequentiell mit Hilfe der für die Datenquelle optimierten binären Klassifikatoren (neben Arzneimittel, positive Arzneimittelwirkung, zusätzlich Indikation, beabsichtigter Effekt und Begleiterkrankung) analysiert und falls ein positives Ergebnis festgestellt wird, die Satzteile der Mitteilung durch die jeweiligen Span-Klassifikatoren identifiziert. Die identifizierten medizinischen Terme werden in einen medizinischen Standardbegriff (z.B. MedDRA Preferred Term) überführt. Das Arzneimittel mit einem Standardproduktwörterbuch (z.B. ATC) abgeglichen. Alle identifizierten Mitteilungsbestandteile werden inklusive zusätzlicher Informationen (z.B. Scoring) gespeichert und können zur weiteren Auswertung herangezogen werden.

Für den Fall, dass kein Arzneimittel identifiziert werden kann oder dass keine Aussage über eine positive Wirkung eines Arzneimittels und/oder über eine Verbesserung eines Gesundheitszustands einer Person identifiziert werden kann, wird das Verfahren abgebrochen und es kann eine Ergebnismeldung mit allen identifizierten Daten ausgegeben oder in einem Logbuch gespeichert werden.

Für den Fall, dass sowohl ein Arzneimittel identifiziert werden kann als auch eine Aussage über eine positive Wirkung eines Arzneimittels und/oder über eine Verbesserung eines Gesundheitszustands einer Person identifiziert werden kann, wird ein dritter Schritt initiiert. Die Weiterverarbeitung kann auch auf bestimmte Arzneimittel (z.B. eines pharmazeutischen Herstellers), auf bestimmte Wirkstoffe (herstellerunabhängig) oder Substanzklassen beschränkt sein. Positive Arzneimittelwirkungen können auch auf bestimmte MedDRA Hierarchiebenen oder Datenquellen (z. B. wissenschaftliche Literatur, Meldungen von medizinischem Personal) beschränkt werden.

In dem dritten Schritt wird geprüft, ob eine Indikation identifiziert werden kann, die mit der positiven Wirkung und/oder der Verbesserung des Gesundheitszustands in einem Zusammenhang steht. Wie beschrieben, werden dazu identifizierte Indikationen und positive Arzneimittelwirkungen verwendet.

Für den Fall, dass eine Indikation identifiziert werden kann, die mit der positiven Wirkung und/oder der Verbesserung des Gesundheitszustands in einem Zusammenhang steht, wird ein vierter Schritt initiiert.

Für den Fall, dass keine Indikation identifiziert werden kann, die mit der positiven Wirkung und/oder der Verbesserung des Gesundheitszustands in einem Zusammenhang steht, kann das Verfahren abgebrochen werden, oder es kann in einem weiteren Schritt eine Ergebnismitteilung mit allen identifizierten Daten ausgegeben oder in einem Logbuch gespeichert werden, wobei die Ergebnismitteilung eine Information beinhaltet, dass für das identifizierte Arzneimittel eine positive Wirkung und/oder über eine Verbesserung eines Gesundheitszustands einer Person identifiziert werden konnte und diese positive Wirkung und/oder diese Verbesserung des Gesundheitszustands im Hinblick auf eine mögliche neue Anwendung für das Arzneimittel zu untersuchen sind.

In dem vierten Schritt wird geprüft, ob die identifizierte Indikation eine Indikation ist, für die das identifizierte Arzneimittel bereits zugelassen und/oder entwickelt wird.

Ergibt diese Prüfung, dass es sich um eine für das Arzneimittel bereits bekannte Indikation handelt, kann das Verfahren abgebrochen werden und es kann eine Ergebnismeldung mit allen identifizierten Daten ausgegeben oder in einem Logbuch gespeichert werden.

Ergibt die Prüfung, dass es sich bei der identifizierten Indikation um keine für das identifizierte Arzneimittel bekannte Indikation handelt, kann ein fünfter Schritt initiiert werden.

In dem fünften Schritt kann eine Ergebnismitteilung über das Vorliegen eines Hinweises auf eine neue Indikation für das Arzneimittel mit allen identifizierten Daten ausgegeben oder in einem Logbuch gespeichert werden.

Die vorliegende Erfindung kann ganz oder teilweise mit einem Computersystem ausgeführt werden. Ein "Computersystem" ist ein System zur elektronischen Datenverarbeitung, das mittels programmierbarer Rechenvorschriften Daten verarbeitet. Ein solches System umfasst üblicherweise eine Steuer- und Recheneinheit, oftmals auch als "Computer" bezeichnet, diejenige Einheit, die einen Prozessor zur Durchführung logischer Operationen und einen Arbeitsspeicher zum Laden eines Computerprogramms umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Joystick, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem (zum Bespiel zur Steuerung durch einen Nutzer) erfolgen über Eingabemittel wie beispielsweise eine Tastatur, eine Maus, ein Mikrofon, ein berührungsempfindliches Display und/oder dergleichen. Ausgaben erfolgen über eine oder mehrere Ausgabeeinheiten, die insbesondere ein Monitor (Bildschirm), ein Drucker und/oder ein Datenspeicher sein kann/können.

Fig. 10 zeigt schematisch und beispielhaft eine Ausführungsform des erfindungsgemäßen Computersystems. Das Computersystem (10) umfasst eine Empfangseinheit (11), eine Steuer- und Recheneinheit (12) und eine Ausgabeeinheit (13).

Das erfindungsgemäße Computersystem (10) ist konfiguriert, mindestens eine Mitteilung über mindestens ein Arzneimittel zu empfangen, und diese Mitteilung zu analysieren, um eine mögliche neue Anwendung für das Arzneimittel zu identifizieren.

Die Steuer- und Recheneinheit (12) dient der Steuerung der Empfangseinheit (11) und der Ausgabeeinheit (13), der Koordinierung der Daten- und Signalflüsse zwischen den verschiedenen Einheiten sowie der Prozessierung von Mitteilungen und weiteren Daten.

Die Empfangseinheit (11) dient zum Empfang von mindestens einer Mitteilung. Die mindestens eine Mitteilung kann beispielsweise über ein Netzwerk (nicht in Fig. 10 dargestellt) von einem anderen Computersystem bereitgestellt / übermittelt und/oder aus einer Datenbank, die ein Bestandteil des erfindungsgemäßen Computersystems sein kann oder mit diesem über ein Netzwerk verbunden sein kann, ausgelesen werden.

Die Übermittlung der mindestens einen Mitteilung kann über eine Netzwerkverbindung oder eine direkte Verbindung erfolgen. Die Übermittlung der mindestens einen Mitteilung kann über eine Funkverbindung (WLAN, Bluetooth, Mobilfunk und/oder dergleichen) und/oder kabelgebunden erfolgen. Es ist denkbar, dass mehrere Empfangseinheiten vorhanden sind.

Von der Empfangseinheit werden die mindestens eine Mitteilung und ggf. weitere Daten an die Steuer- und Recheneinheit übermittelt.

Die Steuer- und Recheneinheit ist konfiguriert, die Mitteilung zu analysieren, um eine mögliche neue Anwendung für ein Arzneimittel zu identifizieren. Es ist denkbar, dass mehrere Steuer- und/oder Recheneinheiten vorhanden sind, die verschiedene Schritte der Analyse vornehmen können.

Über die Ausgabeeinheit (13) kann eine Ergebnismitteilung über das Ergebnis der Analyse angezeigt werden (zum Beispiel auf einem Monitor), ausgegeben werden (z.B. über einen Drucker) oder in einem Datenspeicher gespeichert werden. Es ist denkbar, dass mehrere Ausgabeeinheiten vorhanden sind.

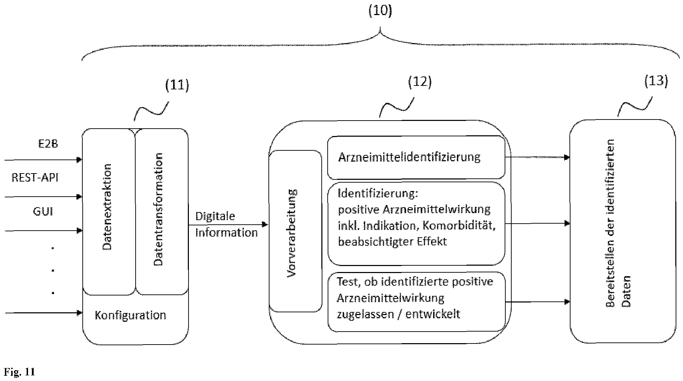

Fig. 11 zeigt schematisch und beispielhaft eine bevorzugte Ausführungsform und Konfiguration des erfindungsgemäßen Computersystems (10) umfasst eine Empfangseinheit (11), eine Steuer- und Recheneinheit (12) und eine Ausgabeeinheit (13).

## Patentansprüche

1. Computer-implementiertes Verfahren umfassend die Schritte:
- Empfangen einer Mitteilung über einen Gesundheitszustand einer Person und/oder über eine Änderung eines Gesundheitszustands der Person und/oder über eine Wirkung eines Arzneimittels und/oder über ein Ausbleiben einer Wirkung des Arzneimittels und/oder über eine Therapie
- Einordnen der Mitteilung in eine von mindestens zwei Klassen,
∘ wobei die mindestens zwei Klassen eine erste Klasse umfasst,
∘ wobei die Mitteilung in die erste Klasse eingeordnet wird, wenn die Mitteilung eine Aussage über eine Verbesserung des Gesundheitszustands der Person enthält und/oder wenn die Mitteilung eine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird,
- für den Fall, dass die Mitteilung in die erste Klasse eingeordnet wurde: Ausgeben der Mitteilung und/oder Übertragen der Mitteilung und/oder Speichern der Mitteilung.

2. Verfahren gemäß Anspruch 1, ferner umfassend den Schritt:
- Identifizieren eines Arzneimittels in der Mitteilung.

3. Verfahren gemäß einem der Ansprüche 1 oder 2, ferner umfassend die Schritte:
- Identifizieren einer Indikation in der Mitteilung, wobei die Indikation mit der Verbesserung des Gesundheitszustands der Person und/oder der gesundheitsfördernden und/oder heilenden und/oder symptomlindernden Wirkung des Arzneimittels und/oder der Therapie in einem Zusammenhang steht,
- Vergleichen der identifizierten Indikation mit Indikationen, für die das Arzneimittel eingesetzt und/oder entwickelt wird,
- für den Fall, dass die identifizierte Indikation mit keiner Indikation, für die das Arzneimittel eingesetzt und/oder entwickelt wird, übereinstimmt: Ausgeben einer Mitteilung über das Vorliegen eines Hinweises auf eine neue Indikation für das Arzneimittel.

4. Verfahren gemäß einem der Ansprüche 1 bis 3, wobei das Einordnen der Mitteilung in eine von mindestens zwei Klassen die folgenden Schritte umfasst:
- Attribuieren von Wörtern und/oder Token und/oder Phrasen in der Mitteilung mit Hilfe eines Wörterbuchs, in dem für einzelne Wörtern und/oder Token und/oder Phrasen ein Attribut verzeichnet ist, das angibt, ob dem jeweiligen Wort und/oder dem Token und/oder der Phrase um eine positive oder negative Bedeutung zukommt,
- Ermitteln eines Attributs für die Mitteilung auf Basis der Attribute der einzelnen Wörter und/oder Token und/oder Phrasen,
- Klassifizieren der Mitteilung anhand des Attributs der Mitteilung.

5. Verfahren gemäß einem der Ansprüche 1 bis 3, wobei das Einordnen der Mitteilung in eine von mindestens zwei Klassen die folgenden Schritte umfasst:
- Zuführen der Mitteilung einem Klassifikationsmodell, wobei das Klassifikationsmodell in einem maschinellen Lernen anhand von Trainingsdaten trainiert worden ist, Mitteilungen in eine von mindestens zwei Klassen einzuordnen, wobei die mindestens zwei Klassen eine erste Klasse umfasst, wobei eine Mitteilung in die erste Klasse eingeordnet wird, wenn die Mitteilung eine Aussage über eine Verbesserung eines Gesundheitszustands einer Person enthält und/oder wenn die Mitteilung eine Aussage über ein Arzneimittel und/oder eine Therapie enthält, in der dem Arzneimittel und/oder der Therapie eine gesundheitsfördernde und/oder heilende und/oder symptomlindernde Wirkung zugesprochen wird,
- Empfangen einer Information von dem Klassifikationsmodell, wobei die Information angibt, in welche Klasse das Klassifikationsmodell die Mitteilung eingeordnet hat.

6. Verfahren gemäß einem der Ansprüche 1 bis 5, wobei die Mitteilung in eine von zwei Klassen eingeordnet wird, wobei die Mitteilung in die zweite Klasse eingeordnet wird, wenn die Mitteilung keine Aussage über eine Verbesserung des Gesundheitszustands der Person enthält und/oder keine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird.

7. Verfahren gemäß einem der Ansprüche 1 bis 5, wobei die Mitteilung in eine von drei Klassen eingeordnet wird, wobei die Mitteilung in die zweite Klasse eingeordnet wird, wenn die Mitteilung eine Aussage über eine Verschlechterung des Gesundheitszustands der Person enthält und/oder eine Aussage über ein Arzneimittel und/oder eine Therapie enthält, in der dem Arzneimittel und/oder der Therapie eine gesundheitsabträgliche und/oder keine symptomlindernde oder eine ausbleibende Wirkung zugesprochen wird, und wobei die Mitteilung in die dritte Klasse eingeordnet wird, wenn sie weder in die erste noch in die zweite Klassen eingeordnet werden kann.

8. Verfahren gemäß einem der Ansprüche 1 bis 7, **dadurch gekennzeichnet, dass** das Verfahren einen oder mehrere der nachfolgend aufgeführten bedingten Schritte umfasst:
- nur dann, wenn die Mitteilung in die erste Klasse eingeordnet wurde, wird ermittelt, welche Stelle / an welchen Stellen in der Mitteilung die Einordnung verursacht haben und die Stelle /die Stellen werden ausgegeben,
- nur dann, wenn die Mitteilung ein Arzneimittel umfasst, wird ermittelt, an welcher Stelle / an welchen Stellen in der Mitteilung das Arzneimittel direkt oder indirekt genannt wird und die Stelle /die Stellen werden ausgegeben,
- nur dann, wenn die Mitteilung eine Indikation umfasst, wird ermittelt, an welcher Stelle / an welchen Stellen in der Mitteilung das Arzneimittel direkt oder indirekt genannt wird und die Stelle /die Stellen werden ausgegeben,
- nur dann, wenn die Mitteilung in die erste Klasse eingeordnet wurde, wird geprüft, ob die Mitteilung ein Arzneimittel und/oder eine Indikation umfasst,
- nur dann, wenn die Mitteilung in ein Arzneimittel umfasst, wird geprüft, ob die Mitteilung eine Indikation umfasst,
- nur dann, wenn die Mitteilung in die erste Klasse eingeordnet wurde und die Mitteilung ein Arzneimittel umfasst, wird geprüft, ob die Mitteilung eine Indikation umfasst.

9. Computersystem, umfassend
• eine Eingabeeinheit,
• eine Steuer- und Recheneinheit und
• eine Ausgabeeinheit,
- wobei die Steuer- und Recheneinheit konfiguriert ist, die Eingabeeinheit zu veranlassen, eine Mitteilung zu empfangen, wobei die Mitteilung eine Aussage über einen Gesundheitszustand einer Person und/oder über eine Änderung eines Gesundheitszustands der Person und/oder über eine Wirkung eines Arzneimittels und/oder über ein Ausbleiben einer Wirkung des Arzneimittels und/oder über eine Therapie umfasst
- wobei die Steuer- und Recheneinheit konfiguriert ist, die Mitteilung in eine von mindestens zwei Klassen einzuordnen, eine erste Klasse und eine zweite Klasse, wobei die Mitteilung in die erste Klasse eingeordnet wird, wenn die Mitteilung eine Aussage über eine Verbesserung des Gesundheitszustands der Person enthält und/oder wenn die Mitteilung eine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird,
- wobei die Steuer- und Recheneinheit konfiguriert ist, die Ausgabeeinheit zu veranlassen, die Mitteilung auszugeben und/oder zu übertragen und/oder zu speichern, wenn die Mitteilung in die erste Klasse eingeordnet wurde.

10. Computersystem gemäß Anspruch 9, wobei das Computersystem konfiguriert ist, einen oder mehrere der in den Ansprüchen 1 bis 8 genannten Schritte auszuführen.

11. Computerlesbares Speichermedium, umfassend Befehle, die bei der Ausführung durch ein Computersystem dieses veranlassen, die folgenden Schritte auszuführen:
- Empfangen einer Mitteilung über einen Gesundheitszustand einer Person und/oder über eine Änderung eines Gesundheitszustands der Person und/oder über eine Wirkung eines Arzneimittels und/oder über ein Ausbleiben einer Wirkung des Arzneimittels und/oder über eine Therapie
- Einordnen der Mitteilung in eine von mindestens zwei Klassen,
∘ wobei die mindestens zwei Klassen eine erste Klasse umfasst,
∘ wobei die Mitteilung in die erste Klasse eingeordnet wird, wenn die Mitteilung eine Aussage über eine Verbesserung des Gesundheitszustands der Person enthält und/oder wenn die Mitteilung eine Aussage über das Arzneimittel und/oder die Therapie enthält, in der dem Arzneimittel und/oder der Therapie eine gesundheitsfördernde und/oder heilende und/oder symptomlindernde Wirkung zugesprochen wird,
- für den Fall, dass die Mitteilung in die erste Klasse eingeordnet wurde: Ausgeben der Mitteilung und/oder Übertragen der Mitteilung und/oder Speichern der Mitteilung.

12. Speichermedium gemäß Anspruch 11, wobei die Befehle das Computersystem veranlassen, einen oder mehrere der in den Ansprüchen 1 bis 8 genannten Schritte auszuführen.
